# EUROPÄISCHE PATENTSCHRIFT

(11) **EP 4 013 360 B1**
(45) Veröffentlichungstag und Bekanntmachung des Hinweises auf die Patenterteilung: **20.03.2024**
(21) Anmeldenummer: 20754728.2
(22) Anmeldetag: 11.08.2020
(51) Int. Cl.: A61F 5/01, A61F 2/64, A61F 2/50

(54) **SYSTEM AUS FEDERADAPTER UND ORTHOPÄDIETECHNISCHE GELENKEINRICHTUNG**
SYSTEM CONSISTING OF SPRING ADAPTER AND ORTHOPEDIC JOINT SYSTEM
SYSTÈME CONSTITUÉ D'UN ADAPTATEUR À RESSORT ET D'UN SYSTÈME D'ARTICULATION ORTHOPÉDIQUE

(30) Priorität: 13.08.2019 DE 102019121797
(43) Veröffentlichungstag der Anmeldung: 22.06.2022
(73) Patentinhaber: Otto Bock Healthcare Products GmbH, 1110 Wien (AT)
(72) Erfinder: SEIFERT, Dirk, 1070 Wien (AT); HAIDER, Stefan, 1020 Wien (AT)
(74) Vertreter: Gramm, Lins & Partner Patent- und Rechtsanwälte PartGmbB
(86) Internationale Anmeldenummer: PCT/EP2020/072444
(87) Internationale Veröffentlichungsnummer: WO 2021/028410

(56) Entgegenhaltungen:
- EP-A1- 3 089 711
- EP-A1- 3 520 741
- WO-A1-2019/076664
- DE-A1-102010 050 318
- DE-A1-102013 011 382
- US-A- 5 653 680
- US-A1- 2010 160 844
- US-A1- 2012 184 880
- US-B2- 8 828 095

## Beschreibung

Die Erfindung betrifft ein System aus einem Federadapter zur Anordnung an einer orthopädietechnischen Gelenkeinrichtung und der orthopädietechnischen Gelenkeinrichtung mit einem Oberteil und einem gelenkig um eine Schwenkachse daran befestigten Unterteil. Die Gelenkeinrichtung weist eine Widerstandseinrichtung auf, die eine Verschwenkung um die Schwenkachse mit einem Widerstand beaufschlagt.

Orthopädietechnische Gelenkeinrichtungen werden in Prothesen, Orthesen und als Sonderfall der Orthesen in sogenannten Exoskeletten eingesetzt. Orthopädietechnische Gelenkeinrichtungen ersetzen in Prothesen nicht vorhandene oder nicht mehr vorhandene Gelenke. In Orthesen werden die Gelenkfunktionen, insbesondere die Verschwenkbarkeit zweier Gliedmaßen zueinander, durch die Verschwenkbarkeit eines Oberteils und eines Unterteils um eine Schwenkachse nachgebildet. Die jeweilige Schwenkachse kann im einfachsten Fall durch eine einachsige, stationäre Gelenkachse ausgebildet sein, alternativ dazu sind polyzentrische Gelenkeinrichtungen möglich und vorgesehen, bei denen eine instationäre Position einer Schwenkachse vorhanden ist. Den orthopädietechnischen Gelenkeinrichtungen sind häufig zur Beeinflussung der Verschwenkbewegung Widerstandseinrichtungen zugeordnet, mit denen die Flexionsbewegung und/oder Extensionsbewegung des Oberteils relativ zu dem Unterteil beeinflusst werden können. Die Widerstandseinrichtungen können mit Federelementen kombiniert sein, beispielsweise um eine Extensionsbewegung nach einer vorhandenen Flexionsbewegung zu unterstützen. Diesen Federelementen können Aktuatoren zugeordnet sein, um eine zeitgerechte Abgabe der gespeicherten Energie zu ermöglichen.

Um das Verschwenkverhalten der orthopädietechnischen Gelenkeinrichtung zu beeinflussen, werden hydraulische, pneumatische und elastische Federelemente eingesetzt, wobei die Federelemente fest eingebaut sind und zur Aufbringung einer Rückstellkraft und/oder Speicherung von Energie dienen. Die hydraulischen und pneumatischen sowie elastischen Elemente sind aufeinander abgestimmt und darauf ausgelegt, die alltäglichen Belastungen aufzunehmen und das übliche Belastungs- und Bewegungsspektrum abzudecken.

Weiterhin gibt es orthopädietechnische Komponenten, die für Spezialanwendungen konstruiert sind. Solche sogenannten Sportprothesen oder Sportorthesen sind für sportliche Belastungen ausgelegt und weisen Feder-Dämpfer-Elemente auf, die einen großen Bewegungsumfang unterstützen und hohen Belastungen standhalten können. Dabei kommen beispielsweise Luftfedern oder auch seilartige Elastomerelemente als Federkomponenten zum Einsatz. Solche Systeme sind nicht für den alltäglichen Gebrauch geeignet und sind in der Regel Einzelanfertigungen, die sehr kostenintensiv sind.

Die DE 10 2010 050 318 A1 betrifft eine Gelenkeinrichtung für eine Prothese oder Orthese mit einem Oberteil und einem verschwenkbar dazu angeordneten Unterteil, wobei zwischen dem Oberteil und dem Unterteil zumindest eine Dämpfereinrichtung angeordnet ist, die während einer Flexion der Gelenkeinrichtung wirksam ist. Die Dämpfereinrichtung ist als ein komprimierbares Elastomerelement ausgebildet.

Die US 2010/0 160 844 A1 betrifft eine orthopädietechnische Gelenkeinrichtung mit einem proximalen Teil und einem distalen Teil mit einer ersten Bremseinrichtung, die eine Bremskraft während einer Flexions- und/oder Extensionsbewegung bereitstellt. Eine zusätzliche Reibungsbremse ist mit der ersten Bremseinrichtung dergestalt gekoppelt, dass während der Flexionsbewegung sowohl die erste Bremseinrichtung als auch die Reibungsbremse eine Bremskraft bereitstellt.

Die US 8 828 095 B2 betrifft Prothesenfuß oder eine Fußorthese mit zwei Teilen, die verschwenkbar aneinander gelagert sind, wobei das zweite Teil einen gewissen Bewegungswiderstand aufweist. Eine Sensoranordnung misst Parameter, die gegenwärtige Betriebsbedingungen anzeigt. Eine Steuerung erzeugt Steuerungssignale in Abhängigkeit von festgestellten Betriebsbedingungen. Eine steuerbare hydraulische Dämpferanordnung ist ausgebildet, um den Bewegungszustand zwischen den beiden Teilen zu verändern.

Die DE 10 2013 011 382 A1 betrifft ein Prothesengelenk mit einem Grundkörper, der eine Aufnahme für eine proximale Komponente sowie eine Lagerstelle für eine schwenkbar an dem Grundkörper angeordnete distale Komponente aufweist. Der Grundkörper weist zudem auf die distale Komponente einwirkende Anschläge oder Federelemente auf. An dem Grundkörper ist zumindest ein Aufnahmeelement lösbar befestigt, in dem oder an dem ein mit der distalen Komponente wechselwirkendes Funktionselement angeordnet ist. Das Funktionselement kann als Feder, Federpaket, Dämpfer, Sensor oder Anschlag ausgebildet sein.

Die EP 3 520 741 A1 betrifft ein Verfahren zur Steuerung einer orthopädietechnischen Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit umgewandelt und in zumindest einem Energiespeicher gespeichert wird. Zur Unterstützung der Relativbewegung wird die gespeicherte Energie wieder umgewandelt und der Gelenkeinrichtung zeitversetzt zugeführt. Eine hydraulische Dämpfereinrichtung ist zwischen dem Oberteil und dem Unterteil angeordnet. Die Dämpfung wird bei unterschiedlichen Gehgeschwindigkeiten unterschiedlich verändert.

Die US 5 653 680 A betrifft eine Handgelenksorthese mit einem ersten Teil, das die Hand umfasst und einem zweiten Teil, das das Handgelenk umfasst sowie einem Gelenk zwischen den beiden Teilen. Zwischen den beiden Teilen sind zumindest eine hydraulische Dämpfereinrichtung und eine Feder angeordnet.

Die WO 2015/101417 A1 betrifft ein Prothesenkniegelenk mit einem Oberteil und einem Unterteil, die über ein viergliedriges Gelenksystem verschwenkbar aneinander gelagert sind. An einem distalen Gelenkglied des Gelenksystems ist ein Federelement angeordnet, dass sich an einem proximalen Gelenkglied des Gelenksystems abstützt. Ein Fluiddämpfer kann eine Dämpfung der Verschwenkbewegung bewirken.

Die WO 2019/076664 A1 betrifft ein orthopädietechnisches Gelenk mit einem Oberteil und einem daran um eine Schwenkachse schwenkbar gelagerten Unterteil mit einer Rotationshydraulik mit einem Schwenkkolben zur Bereitstellung eines hydraulischen Widerstandes bei einer Extension oder Flexion. Eine Vorspanneinrichtung unterstützt eine Verschwenkbewegung des Oberteils relativ zu dem Unterteil und ist über eine Stütze direkt mit dem Schwenkkolben gekoppelt.

Die US 2012/01 84880 A1 betrifft ein Unterstützungssystem für einen Arm eines Nutzers mit einem Geschirr, das an dem Rumpf des Nutzers angelegt wird. Eine Armstütze ist gelenkig an dem Geschirr befestigt. Ein oder mehrere Kompensationselemente sind mit der Armstütze gekoppelt, um zumindest teilweise eine Gravitationskraft, die auf den Arm wirkt, aufzuheben, während sich der Nutzer bewegt und die Armstütze der Bewegung folgt. Wahlweise sind mechanische, pneumatische, hydraulische oder elektrische Elemente zur Dämpfung der Verschwenkbewegung vorgesehen.

Aufgabe der vorliegenden Erfindung ist es daher, einem Patienten, der auf die Nutzung orthopädietechnischer Gelenkeinrichtungen angewiesen ist, einen erweiterten Einsatzbereich der orthopädietechnischen Gelenkeinrichtung zu ermöglichen, insbesondere um mit den in der Regel individuell angepassten orthopädietechnischen Gelenkeinrichtungen Sport treiben zu können.

Erfindungsgemäß wird diese Aufgabe durch ein System mit einer orthopädietechnischen Gelenkeinrichtung mit einem Federadapter mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das System aus einem Federadapter zur Anordnung an einer orthopädietechnischen Gelenkeinrichtung und der orthopädietechnischen Gelenkeinrichtung mit einem Oberteil und einem gelenkig um eine Schwenkachse daran befestigten Unterteil, wobei die Gelenkeinrichtung eine Widerstandseinrichtung aufweist, die eine Verschwenkung um die Schwenkachse mit einem Widerstand beaufschlagt, sieht vor, dass der Federadapter zumindest ein Druckfederelement, Biegefederelement, Zugfederelement oder Torsionsfederelement aufweist, das bei einer Verschwenkung des Oberteils relativ zu dem Unterteil gespannt wird, je nach Ausgestaltung des Federelementes komprimiert, gebogen, gezogen oder tordiert wird. Mit dem Federadapter, der als ein parallel-elastisches Element zu den übrigen Komponenten der Gelenkeinrichtung ausgebildet ist, ist es möglich, die Funktionalität der konventionellen Prothesen oder ,Orthesen zu erweitern, insbesondere bei sportlichen Aktivitäten wie Skifahren oder dergleichen, sodass durch das zusätzliche Anordnen des Federadapters an der orthopädietechnischen Gelenkeinrichtung kostengünstig das Einsatzgebiet der orthopädietechnischen Einrichtung für den jeweiligen Nutzer vergrößert werden kann. Gleichzeitig bleibt die orthopädietechnische Gelenkeinrichtung für die Alltagsversorgung unverändert, sodass sich eine preiswerte und für den Patienten vorteilhafte Nutzungssituation realisieren lässt. Die Widerstandseinrichtung weist ein Gehäuse, z.B. ein Dämpfergehäuse, auf, in dem ein Kolben eine Fluidkammer oder einen Zylinder in eine Flexionskammer und eine Extensionskammer unterteilt. Ist die Widerstandseinrichtung als hydraulischer Lineardämpfer ausgebildet, weist sie eine aus dem Dämpfergehäuse herausragende Kolbenstange auf. Der Federadapter weist eine Befestigungseinrichtung zur Festlegung an der Gelenkeinrichtung und/oder der Widerstandseinrichtung auf, z.B. an der Kolbenstange. Bei einer Anordnung des Federadapters an der Kolbenstange werden der Federadapter und die Druckfeder oder mehrere Druckfederelemente einerseits den zueinander beweglichen Komponenten zugeordnet und andererseits geführt und fixiert, sodass eine Stabilisierung des gesamten Federadapters erreicht wird. Mehrere Druckfedern sind um die Kolbenstange herum angeordnet sein, beispielsweise symmetrisch oder mit unterschiedlichen Abständen zwischen sich um diese herum angeordnet oder achsversetzt dazu neben der Kolbenstange angebracht. Ebenso ist es möglich, dass das Druckfederelement oder mehrere Druckfederelemente koaxial um die Kolbenstange herum angeordnet sind, sodass die Kolbenstange zumindest teilumfänglich von dem Federelement, den Federelementen oder dem Federadapter umgeben wird. Die Kolbenstange wird dann zumindest teilweise von den sie umgebenden Druckfederelementen aufgenommen.

Der Federadapter kann einen distalen Anlageabschnitt und einen proximalen Anlageabschnitt aufweisen, die im montierten Zustand an distalen und proximalen Anlagebereichen der Gelenkeinrichtung und/oder der Widerstandseinrichtung anliegen, zumindest bei einer Kompression anliegen. Bevorzugt liegen die Anlageabschnitte an den Anlagebereichen während der gesamten Nutzung der Gelenkeinrichtung und des Federadapters und des gesamten Nutzungsumfanges der Gelenkeinrichtung an, um ein Spiel, stoßartiges Ineingrifftreten oder störende Klappergeräusche zu vermeiden.

Der Federadapter kann zumindest eine Tubusfeder, Schraubenfeder, Luftfeder, Tellerfeder, ein Tellerfederpaket und/oder ein Elastomerelement aufweisen, um die jeweils benötigte, zusätzliche Federkraft bereitzustellen. Dadurch ist es möglich, einen ausreichenden Widerstand gegen eine außerordentliche und erhöhte Belastung im Bereich einer nicht alltäglichen Nutzung bereitzustellen.

Der Anlageabschnitt, mit dem der Federadapter zwischen zwei beweglichen Komponenten festgelegt werden kann, kann als Teil des Federelementes ausgebildet sein. Alternativ kann der jeweilige Anlageabschnitt an dem Federelement befestigt sein. Das Federelement, z.B. das Druckfederelement oder der Federadapter sind zwischen zwei beweglichen Komponenten der orthopädietechnischen Gelenkeinrichtung angeordnet, beispielsweise zwischen dem Oberteil und dem Unterteil oder im Bereich der Widerstandseinrichtung zwischen einem Gehäuse der Widerstandseinrichtung und dem Oberteil oder einer Aufnahme, z.B. an einer Kolbenstange, wenn die Widerstandseinrichtung als hydraulischer Lineardämpfer ausgebildet ist. Das Federelement oder der gesamte Federadapter können von dem jeweiligen Nutzer oder der Nutzerin des Systems montiert und demontiert werden. Insbesondere Elastomerelemente können aus unterschiedlichen Materialien bestehen und zu Stapeln von Elastomerelementen zusammengesetzt werden, um unterschiedliche Kennlinien zu erzeugen. Beispielsweise ist es möglich, Elastomerelemente als in Radialrichtung geschlitzte Ringscheiben auszubilden, die übereinander gestapelt und beispielsweise um die Kolbenstange oder um eine andere Führung herum angeordnet werden können. Unterschiedliche Elastizitätseigenschaften und Steifigkeiten verschiedener Elastomerscheiben ermöglichen eine einfache Anpassung und Veränderung der Kennlinie des Druckfederelementes oder des Federadapters durch eine geeignete Zusammenstellung der jeweiligen Scheibenpakete.

Der Anlageabschnitt kann eine Halteeinrichtung zur formschlüssigen Festlegung an dem Anlagebereich oder der Gelenkeinrichtung aufweisen, beispielsweise um eine Verdrehung, Verschiebung oder Verkippung des Druckfederelementes oder des gesamten Federadapters relativ zu den übrigen Komponenten der Gelenkeinrichtung zu verhindern. Die Halteeinrichtung kann beispielsweise als Hinterschnitt, Konus, Zapfen oder Vorsprung ausgebildet sein, der in einen entsprechenden Vorsprung oder in eine entsprechende Ausnehmung an oder in dem Haltebereich ausgebildet ist.

Der Federadapter ist vorteilhafterweise auswechselbar und somit abnehmbar und wieder anlegebar an der Gelenkeinrichtung und/oder der Widerstandseinrichtung festgelegt. Dadurch ist es möglich, die Federkennlinie oder die Federsteifigkeit an die jeweiligen Erfordernisse oder Bedürfnisse durch Einsatz des geeigneten Federadapters anzupassen.

Die Befestigungseinrichtung zur Festlegung des Federadapters an der Kolbenstange ist bevorzugt zu einer reversiblen, formschlüssigen Festlegung an der Kolbenstange ausgebildet und kann beispielsweise federnde Komponenten aufweisen, die es ermöglichen, den Federadapter oder die Druckfederelemente unter Aufbringung einer elastischen Umfangskraft an der Kolbenstange oder zwischen dem Oberteil und dem Dämpfergehäuse festzulegen. Es können Scharniere oder Klipseinrichtungen angeordnet oder ausgebildet sein, um den Federadapter oder einzelne Federelemente auszuwechseln und an der Kolbenstange anzubringen.

Eine Weiterbildung der Erfindung sieht vor, dass mehrere Einzelfedern parallel geschaltet in dem Druckfederelement angeordnet sind. Die Einzelfedern selbst können aus in Reihe geschalteten Einzelfederkomponenten bestehen, beispielsweise aus Elastomerscheiben oder dergleichen. Die parallel geschalteten Einzelfedern können unterschiedliche Federcharakteristiken und/oder Eingriffspunkte zur Ausbildung eines abgestuften Federverhaltens aufweisen. So ist es möglich, dass zunächst eine vergleichsweise weiche Feder von Anfang an in Eingriff tritt und eine steifere Feder oder mehrere steifere Federn nach Erreichen eines vorbestimmten Verlagerungsweges komprimiert wird oder werden, um so eine progressive Gesamtfedercharakteristik bereitstellen zu können.

Alternativ oder ergänzend zu den parallel geschalteten Einzelfedern kann der Federadapter zumindest ein Federelement mit seriell geschalteten, unterschiedlichen Federsteifigkeiten aufweist, um einen sich über den Verformungsweg verändernden Widerstand bereitzustellen.

Die Widerstandseinrichtung kann einen hydraulischen und/oder pneumatischen Dämpfer aufweisen, die orthopädietechnische Gelenkeinrichtung ist insbesondere als Prothesengelenk oder Orthesengelenk ausgebildet, insbesondere als ein künstliches Kniegelenk.

Der Federadapter kann verstellbar ausgebildet sein, beispielsweise durch eine Verschiebung der Federkennlinie durch eine Veränderung der Vorspannung oder durch eine Verschiebung des Nullpunktes. Ebenso kann die Steifigkeit veränderlich ausgebildet sein, beispielsweise durch Austausch von Federelementen oder indem die Federlänge und/oder Steifigkeit durch ein Verstellelement oder ein Abstandshalter verändert wird.

Die Offenbarung betrifft ebenfalls einen Federadapter zur lösbaren Befestigung an einer Widerstandseinrichtung, wie oben ausgeführt. Eine Verkapselung mehrerer Druckfederkomponenten oder Druckfederelemente in einem Gehäuse kann vorteilhaft sein. Bei einer Ausgestaltung des Federadapters oder der Druckfederelemente oder Torsionsfederelemente als Elastomer-Elemente können unterschiedliche Formen realisiert werden, wodurch das häufig beschränkte Bauvolumen optimal genutzt werden kann. Die Druckfederelemente oder der Federadapter kann beispielsweise U-förmig ausgebildet sein, gegebenenfalls können die offenen Abschnitte der U-förmigen Druckfederelemente bei übereinander gestapelten Elastomerkomponenten verdreht zueinander angeordnet sein, um eine gleichmäßige Belastung zu erreichen und ein Kippmoment zu verhindern. Die Charakteristiken der Federelemente sind je nach Anwendungsfall auswählbar, sie können linear, degressiv oder progressiv sein.

An dem Federadapter und/oder der Gelenkeinrichtung kann eine Identifiziereinrichtung angeordnet sein, die mit der Widerstandseinrichtung gekoppelt ist. Die Identifiziereinrichtung kann beispielsweise ein RFID-Chip oder ein Sensor oder ein Kontakt sein, der nach dem Anbringen des Federadapters geschlossen wird. Sobald automatisch erkannt ist, dass der Federadapter an der orthopädietechnischen Gelenkseinrichtung angeordnet ist, kann dies zu einem veränderten Dämpferverhalten der Widerstandseinrichtung führen. Beispielsweise kann bei einer elektronisch gesteuerten, veränderbaren Widerstandseinrichtung ein entsprechendes Programm in der Steuerungseinheit abgelegt sein, das eine stärkere oder winkelabhängig angepasste Dämpfung oder eine andere Art und Weise der Verstellung der Widerstände bewirkt. Die Identifiziereinrichtung ermöglicht eine automatische Anpassung des Dämpferverhaltens an die zusätzlichen Federelemente oder aber eine Rückstellung auf die Basiseinstellung nach dem Entfernen des Federadapters.

Neben einem elastischen Verhalten des Federadapters oder der Federelemente kann auch ein geschwindigkeitsproportionales Verhalten über die Viskoseeigenschaften viskoser Elemente oder eines Hydraulikfluids eingestellt werden.

Eine Anbindung an bereits vorhandene Strukturen in der Gelenkeinrichtung kann über demontierbare Achsen, durch Formschluss oder durch Klemmen an bereits vorhandenen Komponenten erreicht werden.

Nachfolgend werden Ausführungsbeispiele der Erfindung anhand der beigefügten Figuren näher erläutert. Es zeigen:
- Figur 1 -: eine schematische Darstellung eines Systems aus Federadapter und Gelenkeinrichtung;
- Figur 2 -: eine schematische Darstellung eines mehrschichtigen Elastomerelementes;
- Figur 3 -: eine schematische Darstellung eines Federadapters mit zwei achsversetzten Federelementen;
- Figur 4 -: eine schematische Darstellung einer Variante der Anordnung des Federadapters;
- Figur 5 -: eine Variante der Figur 4 mit einer Anbindung an dem Unterteil;
- Figur 6 -: eine weitere Variante der Figur 4 mit einer Anbindung an einem Widerstandsgehäuse und einem Oberteil;
- Figur 7 -: eine Variante mit einer Rotationshydraulik;
- Figur 8 -: eine Variante mit einer koaxialen Anbindung des Federadapters; sowie
- Figuren 9-11 -: schematische Darstellungen von Befestigungsmöglichkeiten des Federadapters um eine Kolbenstange.

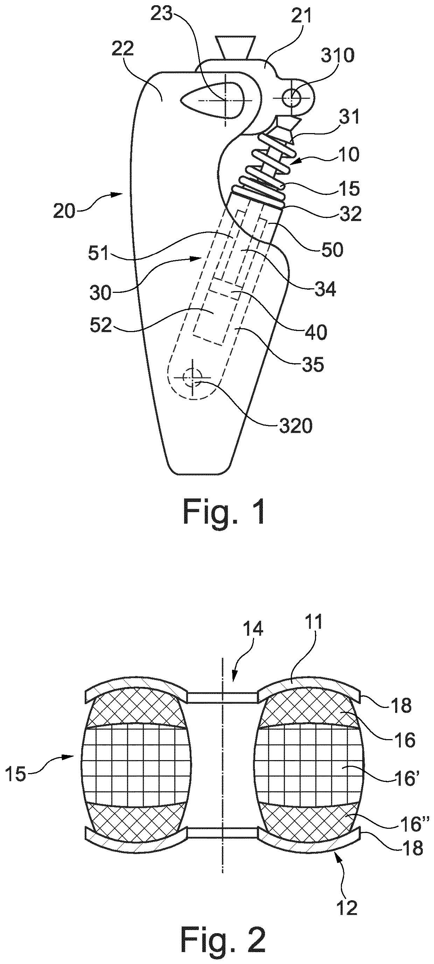

In der Figur 1 ist in einer schematischen Seitenansicht eine orthopädietechnische Gelenkeinrichtung 20 in Gestalt eines Prothesenkniegelenkes dargestellt. Die orthopädietechnische Gelenkeinrichtung 20 weist ein Oberteil 21 und ein daran befestigtes Unterteil 22 auf, die schwenkbar um eine Schwenkachse 23 aneinander gelagert sind. Zwischen dem Oberteil 21 und dem Unterteil 22 ist eine Widerstandseinrichtung 30 angeordnet, die die Verschwenkbewegung um die Schwenkachse 23 beeinflusst. Die Widerstandseinrichtung 30 kann als rein passive Widerstandseinrichtung ausgebildet sein, beispielsweise als ein Fluiddämpfer, insbesondere ein Hydraulikdämpfer. Darüber hinaus kann die Widerstandseinrichtung 30 mit einem magnetorheologischen Fluid versehen sein. Ebenfalls kann ein Aktuator in einer entsprechenden Schaltung einen entsprechenden Widerstand bereitstellen. So kann ein Antrieb im Generatorbetrieb als Widerstandseinrichtung 30 eingesetzt werden. In dem dargestellten Ausführungsbeispiel ist die Widerstandseinrichtung 30 schwenkbar um zwei Lagerachsen 310, 320 gelagert. Der proximale Anlenkpunkt der Widerstandseinrichtung 30 mit der proximalen Lagerachse 310 befindet sich an der in Gehrichtung rückwärtigen Seite des Oberteils 21, die distale Lagerachse 320 ist an dem Unterteil 22 angeordnet oder ausgebildet.

In der Figur 1 ist die orthopädietechnische Gelenkeinrichtung 20 in einer maximal gestreckten oder extendierten Stellung gezeigt. Eine weitere Verschwenkung des Oberteils 21 in Extensionsrichtung, in der dargestellten Konfiguration entgegen dem Uhrzeigersinn, ist nicht mehr möglich. Soll eine Flexion durchgeführt werden, wird das Oberteil 21 im Uhrzeigersinn um die Schwenkachse 23 verschwenkt oder bei einem feststehenden Oberteil 21 das Unterteil 22 entgegen dem Uhrzeigersinn um die Schwenkachse 23 verschwenkt. Die Widerstandseinrichtung 30 in Gestalt eines linear wirkenden Hydraulikdämpfers wird mit einer entsprechenden Kraft beaufschlagt, sodass die obere Lagerachse 310 in Richtung auf die untere Lagerachse 320 bewegt wird. Die Widerstandseinrichtung 30 weist ein Gehäuse 35 auf, in dem ein Kolben 40 an einer aus dem Gehäuse 35 herausragenden Kolbenstange 34 gelagert ist. Der Kolben 40 unterteilt einen in dem Gehäuse 35 ausgebildeten Zylinder 50 in eine Flexionskammer 51 und eine Extensionskammer 52, die strömungstechnisch miteinander verbunden sind. Über ein oder mehrere Ventile, die nicht dargestellt sind, kann der Strömungswiderstand zwischen der Flexionskammer 51 und der Extensionskammer 52 eingestellt und insbesondere in Abhängigkeit von Bewegungszuständen oder Sensordaten verstellt werden. Dazu sind eine Steuerungseinrichtung mit einem Computer oder einem Prozessor mit einer Verstelleinrichtung für die Ventile gekoppelt, sodass in Abhängigkeit von Bewegungsdaten, beispielsweise Verschwenkgeschwindigkeiten, Beschleunigungen, Momenten, Kräften oder dergleichen die Ventile geöffnet oder geschlossen werden können.

An der Widerstandseinrichtung 30 sind Anlagebereiche 31, 32 als Anlageflächen angeordnet oder ausgebildet, zwischen denen ein Federadapter 10 angeordnet ist. Eine distale Anlagefläche 32 ist im Bereich des Gehäuses 35, vorliegend an einem Dämpfergehäuse eines Hydraulikdämpfers angeordnet oder ausgebildet, eine proximale Anlagefläche 31 ist an dem proximalen Ende der Kolbenstange 34 befestigt oder ausgebildet. Die beiden Anlageflächen 31, 32 dienen zur Aufnahme von Kräften, die bei einer Kompression oder Entspannung des Federadapters 10 auftreten. In dem dargestellten Ausführungsbeispiel weist der Federadapter 10 eine Druckfeder 15 in Gestalt einer Schraubenfeder oder Wendelfeder auf, andere Druckfederarten können ebenfalls vorgesehen sein, beispielsweise Elastomerelemente oder dergleichen. Dabei ist es nicht notwendig, dass der Federadapter auch im maximal extendierten Zustand an den beiden Anlageflächen 31, 32 anliegt und eine Druckkraft in Extensionsrichtung ausübt. Es besteht ebenfalls die Möglichkeit, beispielsweise für das Skifahren, dass der Federadapter 10 bei einem eingebeugten Gelenk erst ab Erreichen einer bestimmten Winkelstellung komprimiert wird. Beispielsweise kann sich das in dem Federadapter 10 angeordnete Federelement 15 erst bei einem Beugewinkel von 15° in einer Neutralstellung befinden und erst bei einem weiteren Einbeugen komprimiert werden. Bis zu dem einstellbaren Winkel der Neutralstellung würde eine Zugbelastung zwischen dem Oberteil 21 und dem Unterteil 22 über die Widerstandseinrichtung 30 und den Federadapter 10 ausgeübt werden. Der Federadapter 10 wäre dann zugkräfteübertragend und druckkräfteübertragend zwischen den Anlagebereichen 31, 32 angeordnet.

Statt einer einzelnen Druckfeder 15 können mehrere Federelemente den Federadapter 10 ausbilden oder Teil des Federadapters 10 sein. Verschiedene Federarten können gemischt in einem Federadapter 10 zusammengefasst sein, beispielsweise mehrere Schraubenfedern oder eine Schraubenfeder in Kombination mit einer oder mehreren Tellerfedern oder einem Elastomerelement. Der Federadapter 10 ist lösbar und war leicht auswechselbar an der Widerstandseinrichtung 30 festlegbar ausgebildet, bevorzugt ohne die Widerstandseinrichtung 30 von der Gelenkeinrichtung 20 trennen zu müssen.

In der Figur 2 ist eine Variante des Federadapters 10 in einer Einzeldarstellung gezeigt. Der Federadapter 10 weist an seinem oberen, proximalen Ende einen proximalen Anlageabschnitt 11 und an seinem unteren, distalen Ende einen distalen Anlageabschnitt 12 auf, die in dem dargestellten Ausführungsbeispiel als Teil jeweils eine Tellerfeder 18 oder eines Tellerfederelementes 18 ausgebildet sind.

Zwischen den beiden Tellerfederelementen 18 oder Tellerfedern 18 ist als Druckfederelement 15 eine Kombination mehrerer Elastomerelemente 16, 16', 16" angeordnet. Die Elastomerelemente 16, 16', 16" können aufeinander gestapelt oder miteinander verbunden, beispielsweise verklebt, sein. Die Elastomerelemente oder Federelemente weisen in ihrer Mitte eine Ausnehmung auf, in die beispielsweise die Kolbenstange 34 eines Hydraulikdämpfers eingeführt werden kann. Die Ausnehmung ist so ausgebildet, dass die Kolbenstange 34 zumindest teilweise von dem Federadapter 10 im montierten Zustand umgeben wird. Es kann ebenfalls beispielsweise über eine Scharnierlösung möglich sein, den Federadapter 10 aufzuklappen und um die Kolbenstange herum zu legen. An dem Federadapter 10 kann eine Befestigungseinrichtung 14 angeordnet oder ausgebildet sein, mit der der Federadapter 10 an der Kolbenstange 34 reversibel und formschlüssig festgelegt werden kann. Die Befestigungseinrichtung 14 kann als Riegel, Bolzen, Federelement oder auch Magnet ausgebildet sein, mit dem zwei beweglich zueinander angeordnete Abschnitte oder Bereiche des Federadapters 10 aneinander gehalten werden, um ein ungewolltes Lösen von der Kolbenstange 34 oder der Widerstandseinrichtung 30 insgesamt zu vermeiden. Die Anlageabschnitte 11, 12 sind bevorzugt so ausgebildet, dass sie korrespondierend zu den Anlagebereichen 31, 32 ausgebildet sind, sie können auch in einem formschlüssigen Eingriff mit den Anlagebereichen 31, 32 stehen, um eine Relativbewegung, beispielsweise ein Verdrehen um die Längserstreckung der Widerstandseinrichtung 30 zu verhindern. Die Anlageabschnitte 11, 12 können beispielsweise in die Anlagebereiche 31, 32 einschnappen oder damit verrastet werden.

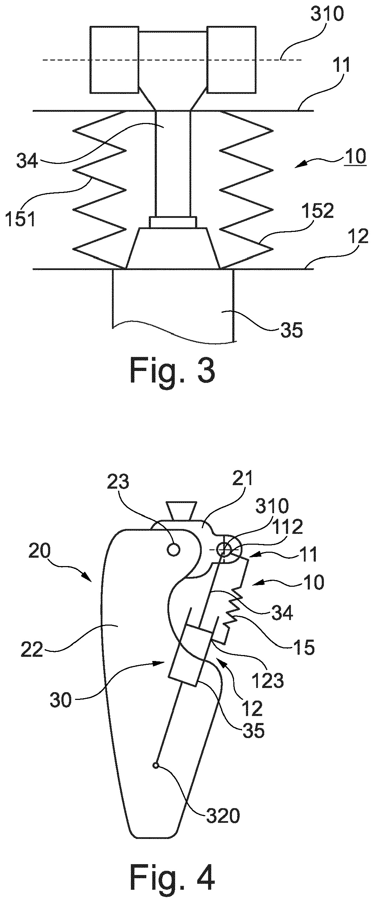

In der Figur 3 ist eine Variante des Federadapters 10 gezeigt, der zwei Anlageabschnitte 11, 12 aufweist, von denen der proximale Anlageabschnitt 11 an einer Verdickung der Kolbenstange 34 anliegt und bei einer Einbeugung des nicht dargestellten Gelenkes und einer Belastung der Kolbenstange 34 mit einer Druckkraft die beiden Federelemente 151, 152 spannt und komprimiert. Das Widerlager dazu ist der distale Anlageabschnitt 12, der sich auf dem Gehäuse 35 der Widerstandseinrichtung 30 abstützt. Es können weitere Federelemente zwischen den beiden Anlageabschnitten 11, 12 angeordnet sein, die weiteren Federelemente können parallel wirksam zu den bereits vorhandenen Federelementen angeordnet sein. Alternativ oder ergänzend dazu können mehrere unterschiedliche Federelemente in Reihe wirksam hintereinander angeordnet sein. Ebenso ist es möglich, dass zusätzliche Federelemente erst nach einem bestimmten Verlagerungsweg der beiden Anlageabschnitte 11, 12 aufeinander zu wirksam werden und komprimiert werden, beispielsweise indem ein Elastomerelement oder ein Tubusdämpfer anfänglich beanstandet zu dem proximalen Anlageabschnitt 11 an dem distalen Anlageabschnitt 12 angeordnet ist.

In der Figur 4 ist eine Variante der Anordnung eines Federadapters 10 an der Gelenkeinrichtung 20 dargestellt. Der proximale Anlageabschnitt 11 ist in dem dargestellten Ausführungsbeispiel an der proximalen Lagerachse 310 der Kolbenstange 34 an dem Oberteil 21 angeordnet. Der distale Anlageabschnitt 12 befindet sich an dem Gehäuse 35 der Widerstandseinrichtung 30, die beispielsweise als ein Hydraulikdämpfer oder Pneumatikdämpfer ausgebildet sein kann. An beiden Anlageabschnitten 11, 12 sind jeweils Halteeinrichtungen 112, 123 angeordnet oder ausgebildet, mit denen der Federadapter 10 formschlüssig an der Gelenkeinrichtung 20 beispielsweise der Widerstandseinrichtung 30 festlegbar ist. Die Halteeinrichtungen 112, 123 können Durchgangsbohrungen für Schrauben oder Bolzen sein, die mit oder ohne Gewinde ausgebildet sein können. Ebenso können die Halteeinrichtungen 112, 123 zum Einstecken und Verrasten in korrespondierende Ausnehmungen an dem Oberteil 21 und dem Gehäuse 35 beispielsweise dem Unterteil 22 ausgebildet sein.

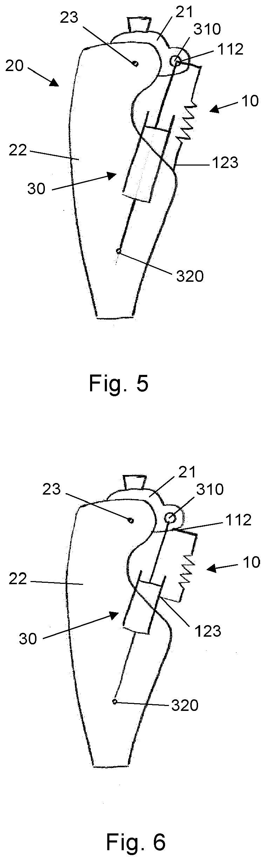

Eine weitere Variante ist in der Figur 5 dargestellt, bei der die proximale Anbindung wie in der Figur 4 ausgeführt ist. Die distale Anbindung erfolgt an dem Unterteil 22 über die distale Halteeinrichtung 123 an dem distalen Anlageabschnitt 12. In der Ausführungsform gemäß Figur 6 erfolgt die distale Anbindung wie in der Figur 4 beschrieben, die proximale Anbindung erfolgt jedoch nicht im Bereich der Lagerachse 310, sondern an der Unterseite des Oberteils 21 über die proximale Halteeinrichtung 112.

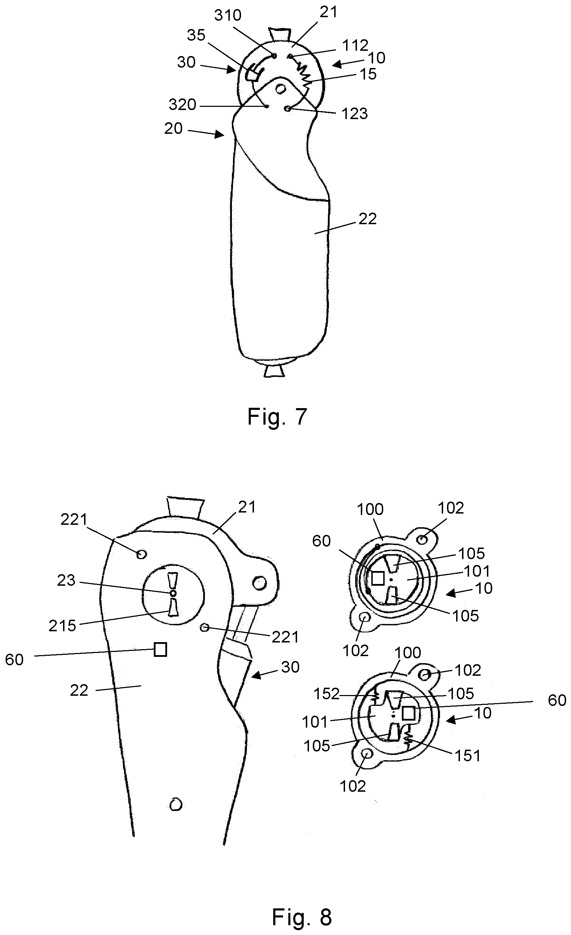

In der Figur 7 ist eine weitere Variante gezeigt, bei der statt einer linear wirksamen Widerstandseinrichtung 30, beispielsweise einem linear wirkenden Hydraulikdämpfer, eine Rotationshydraulik oder rotatorisch wirksame Widerstandseinrichtung 30 vorgesehen ist. Statt einer hydraulischen Dämpfung über Ventile oder Drosseln in einem Strömungskanal kann die Widerstandseinrichtung 30 auch über magnetorheologische Fluide oder Antriebe im Generatorbetrieb realisiert werden. Der Federadapter 10 ist an dem Oberteil 21 und dem Unterteil 22 angeordnet und festgelegt. In dem dargestellten Ausführungseispiel sind die Halteeinrichtungen 112, 123 beispielsweise Zapfen oder Schrauben, die auf einer gleichen Kreisbahn um die Schwenkachse 23 herum angeordnet sind, sodass bei einer Verschwenkung des Oberteils 21 relativ zu dem Unterteil 22 die beiden Halteeinrichtungen 112, 123 aufeinander zu bewegt werden. Grundsätzlich ist es auch möglich, dass die Halteeinrichtungen 112,123 in jeweils einem unterschiedlichen Radius zu der Schwenkachse 23 angeordnet sind, sodass neben einer reinen Druckkraft von dem Federelement 15 auch Scherkräfte, Biegekräfte und/oder bei einer alternativen Ausgestaltung des Federelementes 15 Torsionskräfte auftreten, die durch eine entsprechende Ausgestaltung des Federelementes 10 aufgenommen und gespeichert werden. Der Federadapter 10 kann auch als auf Torsion oder Biegung wirksamer Federadapter ausgebildet sein und eine Torsionsfeder oder eine Biegefeder oder mehrere Federn aufweisen. Ebenso ist es möglich, dass mehrere unterschiedliche Federtypen, beispielsweise Torsionsfeder und Biegefeder, kombiniert werden.

Eine weitere Variante ist in der Figur 8 gezeigt, bei der in der linken Zeichnung die Gelenkeinrichtung 20 mit einer Widerstandseinrichtung 30 und in den rechten Zeichnungen zwei Ausführungsformen des Federadapters 10 dargestellt sind. Der grundsätzliche Aufbau der Gelenkeinrichtung 20 entspricht der der Figuren 1 sowie 4 bis 6. Statt einer Anordnung des Federadapters 10 zwischen zwei sich aufeinander zu bewegenden Anlagebereichen, sieht die Figur 8 eine koaxiale Anbindung des Federadapters 10 vor. In der Gelenkeinrichtung 20 sind zwei Zapfen oder Bohrungen 221 vorgesehen, die korrespondierend zu Bohrungen oder Zapfen 102 an einem ersten Gehäuseteil 100 des Federadapters 10 positioniert sind. Über die Zapfen oder Bohrungen 221, die mit Gewinden versehen sein können, ist es möglich, das erste Gehäuseteil 100 formschlüssig, beispielsweise durch Aufstecken und Verriegeln und/oder durch Verschrauben, an der Gelenkeinrichtung 20 festzulegen. Die Festlegung erfolgt in dem dargestellten Ausführungsbeispiel an dem Unterteil 22. In dem Oberteil 21 sind Formschlusseinrichtung 215 beiderseits der Schwenkachse 23 angeordnet oder ausgebildet. Die Formschlusseinrichtungen können auch ungleich beabstandet von der Schwenkachse 23 an dem Oberteil 21 angeordnet oder ausgebildet sein, ebenso kann auch nur eine Formschlusseinrichtung 215 oder mehr als zwei Formschlusseinrichtungen 215 vorgesehen sein. Die Formschlusseinrichtungen 215 können als Vorsprünge, Vertiefungen, Verzahnungen oder Bohrungen ausgebildet sein, in die korrespondierend geformte Formschlusselemente 105 an einem zweiten, drehbar zu dem ersten Gehäuseteil 100 gelagerten Gehäuseteil 101 im montierten Zustand eingreifen. Zwischen den beiden Gehäuseteilen 100,101 befinden sich in der oben dargestellten Variante des Federadapters 10 eine Spiralfeder oder Torsionsfeder 105, während im unteren Ausführungsbeispiel des Federadapters 10 zwei oder mehr Druckfederelemente 151, 152 angeordnet sind. Wird im montierten Zustand das Oberteil 21 um die Schwenkachse 23 relativ zu dem Unterteil 22 verschwenkt, werden durch die Formschlusselemente 105, 215 relativ zu dem Unterteil 22 und dem ersten Gehäuseteil 100 verdreht, da das erste Gehäuseteil 100 über die formschlüssige Verbindung beispielsweise über Zapfen und Bohrungen 102, 221 ortsfest und nicht drehbeweglich an dem Unterteil festgelegt ist. Das zweite Gehäuseteil 101 verdreht sich relativ zu dem erste Gehäuseteil 100 und die Federelemente 151, 152 oder das Federelement 15 werden oder wird gespannt.

In dem Ausführungsbeispiel gemäß Figur 8 sind sowohl an dem Federadapter 10 als auch an der Gelenkeinrichtung 20 Identifiziereinrichtungen 60 angeordnet, die automatisch das Vorhandensein oder Nichtvorhandensein des Federadapters 10 detektieren. Ist ein Federadapter 10 vorhanden und montiert, wird ein entsprechendes Signal von der Identifizierungseinrichtung 60 zu einer nicht dargestellten Steuerungseinrichtung gesendet, sodass es möglich ist, unterschiedliche Dämpfungseinstellungen oder Widerstandseinstellungen in der Widerstandseinrichtung 30 vorzunehmen. Die Einstellungen können je nach eingesetztem Federadapter 10 unterschiedlich sein. Werden beispielsweise sehr starke Federelemente eingesetzt, um besonders hohe Belastungen wie zum Beispiel beim Skifahren aufzunehmen, kann die Widerstandseinrichtung 30 entsprechend geänderte Widerstände bereitstellen. In einer Ausgestaltung als Hydraulikdämpfer kann der Dämpfungsgrad sowie eine Dämpfungsprogression in Abhängigkeit von dem Federwiderstand durch den Federadapter 10 verstellt werden. Eine Detektion kann über ein RFID- Element, eine magnetische Kennung oder ein anderes Sensorelement erfolgen. Dadurch ist es möglich, dass die orthopädietechnische Gelenkeinrichtung 20 automatisch in Abhängigkeit von dem gewählten Federadapter 10 ein angepasstes Dämpfungsprogramm oder Widerstandsprogramm einstellt.

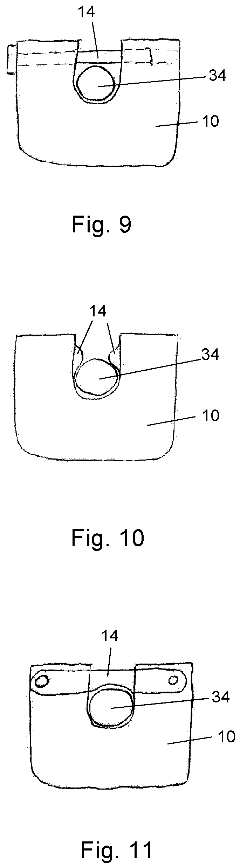

In den Figuren 9 bis 11 sind schematische Schnittdarstellungen zu unterschiedlichen Fixiermöglichkeiten des Federadapters 10 um eine Kolbenstange 34 herum dargestellt. In der Figur 9 erfolgt die Sicherung des Federadapters 10 oder des Federpaketes über einen Bolzen als Befestigungseinrichtung 14. In dem Federpaket des Federadapters 10 ist eine U-förmige Ausnehmung zur Aufnahme der Kolbenstange 34 vorgesehen. Darüber hinaus ist ein Gewinde und ein Durchgangsloch in dem Federadapter 10 angeordnet, sodass nach der Montage des Federadapters 10 um die Kolbenstange 34 herum die Einführöffnung der Ausnehmung durch Einstecken und Verschrauben des Bolzens 14 geschlossen wird und der Federadapter sicher an der Kolbenstange 34 fixiert ist.

In der Figur 10 ist die Befestigungseinrichtung 14 als ein elastischer Arretier-Mechanismus mit elastischen Vorsprüngen aus einem Elastomer oder aus Blattfedern oder dergleichen ausgebildet. Dadurch kann der Federadapter 10 werkzeuglos montiert und demontiert werden.

In der Figur 11 ist die Befestigungseinrichtung 14 als ein Klapphebel ausgebildet, der schwenkbar an dem Federadapter 10 gelagert ist und nach dem Einführen der Kolbenstange 34 verschlossen werden kann.

## Patentansprüche

1. System aus einem Federadapter (10) zur Anordnung an einer orthopädietechnischen Gelenkeinrichtung (20) und der orthopädietechnischen Gelenkeinrichtung (20) mit einem Oberteil (21) und einem gelenkig um eine Schwenkachse (23) daran befestigten Unterteil (22), wobei die Gelenkeinrichtung (20)eine Widerstandseinrichtung (30) aufweist, die eine Verschwenkung um die Schwenkachse (23) mit einem Widerstand beaufschlagt, wobei der Federadapter (10) zumindest ein Druckfederelement (15, 16, 16', 16", 18), Biegefederelement und/oder Torsionsfederelement aufweist und bei einer Verschwenkung des Oberteils (21) relativ zu dem Unterteil (22) gespannt wird, wobei die Widerstandseinrichtung (30) ein Gehäuse (35) aufweist, in dem ein Kolben (40) eine Fluidkammer (50) in eine Flexionskammer (51) und eine Extensionskammer (52) unterteilt, und der Federadapter (10) eine Befestigungseinrichtung (14) zur Festlegung an der Gelenkeinrichtung (20) aufweist und die Widerstandseinrichtung (30) als ein Dämpfer ausgebildet ist, der eine aus dem Gehäuse (35) herausragende Kolbenstange (34) aufweist, an der ein Kolben (40) in dem Gehäuse (30) die Fluidkammer (50) in die Flexionskammer (51) und die Extensionskammer (52) unterteilt und die Befestigungseinrichtung (14) zur Festlegung des Federadapters (10) an der Kolbenstange (34) ausgebildet ist, **dadurch gekennzeichnet, dass** mehrere Druckfedern um die Kolbenstange (34) herum angeordnet sind.

2. System nach Anspruch 1, **dadurch gekennzeichnet, dass** der Federadapter (10) einen distalen Anlageabschnitt (12) und einen proximalen Anlageabschnitt (11) aufweist, die im montierten Zustand an distalen und proximalen Anlagebereichen (31, 32) der Gelenkeinrichtung (20) und/oder der Widerstandseinrichtung (30) bei einer Kompression anliegen.

3. System nach Anspruch 1 oder 2, **dadurch gekennzeichnet, dass** der Federadapter (10) zumindest eine Tubusfeder, Schraubenfeder (17), Luftfeder, Tellerfeder (18) und/oder ein Elastomerelement (16, 16', 16") aufweist.

4. System nach Anspruch 2, **dadurch gekennzeichnet, dass** der Anlageabschnitt (11, 12) als Teil des Federelementes (15) ausgebildet oder daran befestigt ist.

5. System nach Anspruch 2 oder 4, **dadurch gekennzeichnet, dass** der Anlageabschnitt (11, 12) eine Halteeinrichtung (112, 123) zur formschlüssigen Festlegung an der Gelenkeinrichtung (20) aufweist.

6. System nach einem der voranstehenden Ansprüche, **dadurch gekennzeichnet, dass** der Federadapter (10) auswechselbar/abnehmbar an der Gelenkeinrichtung (20) und/oder der Widerstandseinrichtung (30) festgelegt ist.

7. System nach einem der voranstehenden Ansprüche, **dadurch gekennzeichnet, dass** die Befestigungseinrichtung (14) zur reversiblen, formschlüssigen Festlegung des Federadapters (10) an der Kolbenstange (34) oder zwischen dem Oberteil (21) und dem Gehäuse (35) ausgebildet ist.

8. System nach einem der voranstehenden Ansprüche, **dadurch gekennzeichnet, dass** mehrere Einzelfedern (151, 152) parallel geschaltet in dem Federadapter (10) angeordnet sind.

9. System nach Anspruch 8, **dadurch gekennzeichnet, dass** die parallel geschalteten Einzelfedern (151, 152) unterschiedliche Federcharakteristiken und/oder Eingriffspunkte zur Ausbildung eines abgestuften Federverhaltens aufweisen.

10. System nach einem der voranstehenden Ansprüche, **dadurch gekennzeichnet, dass** der Federadapter (10) zumindest ein Federelement mit seriell geschalteten, unterschiedlichen Federsteifigkeiten aufweist.

11. 12. System nach einem der voranstehenden Ansprüche, **dadurch gekennzeichnet, dass** die Widerstandseinrichtung (30) einen hydraulischen und/oder pneumatischen Dämpfer aufweist und die orthopädietechnische Gelenkeinrichtung (20) als Orthesengelenk oder Prothesengelenk ausgebildet ist.

12. System nach einem der voranstehenden Ansprüche, **dadurch gekennzeichnet, dass** der Federadapter (10) verstellbar ausgebildet ist.

13. System nach einem der voranstehenden Ansprüche, **dadurch gekennzeichnet, dass** der Federadapter (10) ein mit dem Oberteil (21) koppelbares erstes Gehäuseteil (101) und ein mit dem Unterteil (22) oder der Widerstandseinrichtung (30) koppelbares zweites Gehäuseteil (100) aufweist, zwischen denen zumindest ein Federelement (151, 152) spannbar angeordnet ist.

14. System nach Anspruch 13, **dadurch gekennzeichnet, dass** an den Gehäuseteilen (100, 101) Formschlusseinrichtungen (105, 215) zur Festlegung an dem Oberteil (21) und dem Unterteil (22) oder der Widerstandseinrichtung (30) angeordnet oder ausgebildet sind.

15. System nach einem der voranstehenden Ansprüche, **dadurch gekennzeichnet, dass** an dem Federadapter (10) und/oder der Gelenkeinrichtung (20) eine Identifiziereinrichtung (60) angeordnet ist, die mit der Widerstandseinrichtung (30) gekoppelt ist.

## Claims

1. A system consisting of a spring adapter (10) for arrangement on an orthopedic joint device (20) and the orthopedic joint device (20) having an upper part (21) and a lower part (22) fastened thereto so as to be pivotable about a pivot axis (23), whereas the joint device (20) having a resistance device (30) that applies a resistance to pivoting about the pivot axis (23), whereas the spring adapter (10) comprises at least one compression spring element (15, 16, 16', 16", 18), bending spring element and/or torsion spring element and is tensioned when the upper part (21) is pivoted relative to the lower part (22), whereas the resistance device (30) comprises a housing (35) in which a piston (40) divides a fluid chamber (50) into a flexion chamber (51) and an extension chamber (52), and wherein the spring adapter (10) comprises a fastening device (14) for securing it to the joint device (20)and the resistance device (30) is embodied as a damper comprising a piston rod (34) which protrudes from the housing (35) and on which a piston (40) divides a fluid chamber (50) in the housing (30) into a flexion chamber (51) and an extension chamber (52), and the fastening device (14) is designed to secure the spring adapter (10) to the piston rod (34), **characterized in that** a plurality of compression springs are arranged around the piston rod (34).

2. The system as claimed in claim 1, **characterized in that** the spring adapter (10) comprises a distal abutment portion (12) and a proximal abutment portion (11) which in the assembled state abut against distal and proximal abutment regions (31, 32) of the joint device (20) and/or the resistance device (30) in the case of a compression.

3. The system as claimed in claim 1 or 2, **characterized in that** the spring adapter (10) comprises at least one tube spring, helical spring (17), air spring, Belleville washer (18) and/or an elastomer element (16, 16', 16").

4. The system as claimed in claim 2, **characterized in that** the abutment portion (11, 12) is formed as part of the spring element (15) or fastened thereto.

5. The system as claimed in claim 2 or 4, **characterized in that** the abutment portion (11, 12) comprises a holding device (112, 123) for securing it to the joint device (20) in interlocking fashion.

6. The system as claimed in any one of the preceding claims, **characterized in that** the spring adapter (10) is secured in interchangeable/removable fashion to the joint device (20) and/or the resistance device (30).

7. The system as claimed in any one of the preceding claims, **characterized in that** the fastening device (14) is designed for reversibly securing the spring adapter (10) to the piston rod (34) in interlocking fashion or between the upper part (21) and the housing (35).

8. The system as claimed in any one of the preceding claims, **characterized in that** a plurality of individual springs (151, 152) are arranged in the spring adapter (10) and connected in parallel.

9. The system as claimed in claim 8, **characterized in that** the individual springs (151, 152) connected in parallel have different spring characteristics and/or different engagement points for forming a gradated spring behavior.

10. The system as claimed in any one of the preceding claims, **characterized in that** the spring adapter (10) comprises at least one spring element with different spring stiffnesses connected in series.

11. The system as claimed in any one of the preceding claims, **characterized in that** the resistance device (30) comprises a hydraulic and/or pneumatic damper and the orthopedic joint device (20) is designed as an orthosis joint or prosthesis joint.

12. The system as claimed in any one of the preceding claims, **characterized in that** the spring adapter (10) has an adjustable form.

13. The system as claimed in any one of the preceding claims, **characterized in that** the spring adapter (10) comprises a first housing part (101) that is capable of being coupled to the upper part (21) and a second housing part (100) that is capable of being coupled to the lower part (22) or the resistance device (30), at least one spring element (151, 152) being arranged between these housing parts so as to be able to be tensioned.

14. The system as claimed in claim 13, **characterized in that** interlocking devices (105, 215) are arranged or formed on the housing parts (100, 101) for the purposes of securing these to the upper part (21) and the lower part (22) or the resistance device (30).

15. The system as claimed in any one of the preceding claims, **characterized in that** an identification device (60) which is coupled to the resistance device (30) is arranged on the spring adapter (10) and/or the joint device (20).

## Revendications

1. Système constitué d'un adaptateur à ressort (10), destiné à être disposé sur un dispositif d'articulation orthopédique (20), et dudit dispositif d'articulation orthopédique (20), comprenant une partie supérieure (21) et une partie inférieure (22) disposée sur celle-ci de manière articulée autour d'un axe de pivotement (23),
dans lequel
le dispositif d'articulation (20) comprend un dispositif de résistance (30) qui impose une résistance à un pivotement autour de l'axe de pivotement (23),
l'adaptateur à ressort (10) comprend au moins un élément de ressort de compression (15, 16, 16', 16", 18), un élément de ressort de flexion et/ou un élément de ressort de torsion et est armé lors d'un pivotement de la partie supérieure (21) par rapport à la partie inférieure (22),
le dispositif de résistance (30) comprend un boîtier (35) dans lequel un piston (40) subdivise une chambre à fluide (50) en une chambre de flexion (51) et en une chambre d'extension (52), et
l'adaptateur à ressort (10) comprend un dispositif de fixation (14) destiné à être fixé sur le dispositif d'articulation (20), et
le dispositif de résistance (30) est conçu comme un amortisseur qui comprend une tige de piston (34) dépassant du boîtier (35), sur laquelle un piston (40) dans le boîtier (30) subdivise la chambre à fluide (50) en la chambre de flexion (51) et en la chambre d'extension (52), et le dispositif de fixation (14) est conçu pour fixer l'adaptateur à ressort (10) sur la tige de piston (34),
**caractérisé en ce que** plusieurs ressorts de compression sont disposés autour de la tige de piston (34).

2. Système selon la revendication 1,
**caractérisé en ce que** l'adaptateur à ressort (10) présente une portion d'appui distale (12) et une portion d'appui proximale (11) qui, à l'état monté, s'appuient sur des zones d'appui distale et proximale (31, 32) du dispositif d'articulation (20) et/ou du dispositif de résistance (30) lors d'une compression.

3. Système selon la revendication 1 ou 2,
**caractérisé en ce que** l'adaptateur à ressort (10) comprend au moins un ressort tubulaire, un ressort hélicoïdal (17), un ressort pneumatique, une rondelle-ressort (18) et/ou un élément en élastomère (16, 16', 16").

4. Système selon la revendication 2,
**caractérisé en ce que** la portion d'appui (11, 12) est conçue comme une partie de l'élément de ressort (15) ou est fixée à celui-ci.

5. Système selon la revendication 2 ou 4,
**caractérisé en ce que** la portion d'appui (11, 12) présente un dispositif de retenue (112, 123) pour la fixation par complémentarité de forme sur le dispositif d'articulation (20).

6. Système selon l'une des revendications précédentes,
**caractérisé en ce que** l'adaptateur à ressort (10) est fixé de manière interchangeable/amovible sur le dispositif d'articulation (20) et/ou sur le dispositif de résistance (30).

7. Système selon l'une des revendications précédentes,
**caractérisé en ce que** le dispositif de fixation (14) est conçu pour fixer l'adaptateur à ressort (10) de manière réversible et par complémentarité de forme sur la tige de piston (34) ou entre la partie supérieure (21) et le boîtier (35).

8. Système selon l'une des revendications précédentes,
**caractérisé en ce que** plusieurs ressorts individuels (151, 152) sont montés en parallèle dans l'adaptateur à ressort (10).

9. Système selon la revendication 8,
**caractérisé en ce que** les ressorts individuels (151, 152) montés en parallèle présentent des caractéristiques d'élasticité et/ou des points d'engagement différents pour avoir un comportement d'élasticité gradué.

10. Système selon l'une des revendications précédentes,
**caractérisé en ce que** l'adaptateur à ressort (10) présente au moins un élément de ressort ayant des raideurs différentes prévues en série.

11. Système selon l'une des revendications précédentes,
**caractérisé en ce que** le dispositif de résistance (30) comprend un amortisseur hydraulique et/ou pneumatique, et le dispositif d'articulation orthopédique (20) est conçu comme une articulation d'orthèse ou une articulation de prothèse.

12. Système selon l'une des revendications précédentes,
**caractérisé en ce que** l'adaptateur à ressort (10) est réalisé de manière réglable.

13. Système selon l'une des revendications précédentes,
**caractérisé en ce que** l'adaptateur à ressort (10) comprend une première partie de boîtier (101) apte à être couplée à la partie supérieure (21) et une deuxième partie de boîtier (100) apte à être couplée à la partie inférieure (22) ou au dispositif de résistance (30), entre lesquelles au moins un élément de ressort (151, 152) est disposé de manière à pouvoir être armé.

14. Système selon la revendication 13,
**caractérisé en ce que** des dispositifs de liaison par complémentarité de forme (105, 215) pour la fixation sur la partie supérieure (21) et la partie inférieure (22) ou le dispositif de résistance (30) sont disposés ou formés sur les parties de boîtier (100, 101).

15. Système selon l'une des revendications précédentes,
**caractérisé en ce qu'**un dispositif d'identification (60) est disposé sur l'adaptateur à ressort (10) et/ou sur le dispositif d'articulation (20), lequel est couplé au dispositif de résistance (30).
